# EUROPEAN PATENT APPLICATION

(11) **EP 3 431 004 A1**
(43) Date of publication of application: **23.01.2019**
(21) Application number: 17182499.8
(22) Date of filing: 21.07.2017
(51) Int. Cl.: A61B 5/145, A61B 5/1455, A61B 5/00

(54) **WEARABLE DEVICE AND METHOD FOR MEASURING A PHYSIOLOGICAL SIGNAL**

(71) Applicant: Koninklijke Philips N.V., 5656 AE Eindhoven (NL)
(72) Inventor: HUIJBREGTS, Laurentia Johanna, 5656 AE Eindhoven (NL); BOURQUIN, Yannyk Parulian Julian, 5656 AE Eindhoven (NL); MENA BENITO, Maria Estrella, 5656 AE Eindhoven (NL)
(74) Representative: de Haan, Poul Erik

(57) **Abstract**

The present invention relates to the fields of medical technology as well as fitness and activity trackers. To provide improved wearing comfort, in particular by reducing skin irritation, a wearable device (10) adapted to be worn by a user (100) is provided, the wearable device (10) comprising a contact sensor (20) adapted to contact a skin of the user (100) and adapted to measure a physiological signal of the user; and a sweat removal device (30) adapted to move sweat away from said contact sensor (20). The present invention further relates to a corresponding method.

## Description

### FIELD OF THE INVENTION

The present invention relates to the fields of medical technology as well as fitness and activity trackers and, in particular, to a wearable device adapted to be worn by a user, the wearable device comprising a contact sensor adapted to contact a skin of the user and adapted to measure a physiological signal of the user. The present invention further relates to a corresponding method.

### BACKGROUND OF THE INVENTION

Vital signs of a person, for example the heart rate (HR), the respiration rate (RR) or the arterial blood oxygen saturation (SpO2), serve as indicators of the current health or fitness state of a person and as powerful predictors for serious medical events. For this reason, vital signs are extensively monitored in inpatient and outpatient care settings, at home or in further health, leisure and fitness settings.

Recently, wearable devices such as activity trackers, sports watches, and health watches have been developed that measure e.g. the heart rate (HR) by using blood volume sensing techniques, such as photoplethysmography (PPG) or bio-impedance.

Plethysmography generally refers to the measurement of volume changes of an organ or a body part and in particular to the detection of volume changes due to a cardiovascular pulse wave travelling through the body of a subject with every heartbeat. Photoplethysmography (PPG) is an optical measurement technique that evaluates a time-varying change of light reflectance or transmission of an area or volume of interest. PPG is based in the principle that blood absorbs light more than surrounding tissue, so that variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation (SpO2) can be determined.

There is a need to ensure that wearable devices can be used to accurately determine vital signs under different conditions.

Wearable devices are usually worn on the arm or wrist. In order to provide reliable measurements, the sensor needs to make good contact with the skin and the device should preferably be strapped quite tightly. One reason why companies of PPG-based heart rate monitors advise their customers to strap the wearable device quite tightly around the arm is because at a higher pressure, the blood volume variations of the pulse of the arterial blood become stronger with respect to other blood volume variations (e.g. in veins and venules), and thereby the signal-to-noise ratio increases. Another reason to advise the customers to strap the device quite tightly is to avoid external light entering underneath the watch, thereby interfering with the signal.

### SUMMARY OF THE INVENTION

It is an object of the present invention to provide a wearable device with improved wearing comfort, in particular reducing skin irritation. It would further be advantageous to provide a wearable device that enables measurements with improved accuracy and/or reliability.

In a first aspect of the present invention a wearable device adapted to be worn by a user is presented, the wearable device comprising:
- a contact sensor adapted to contact a skin of the user and adapted to measure a physiological signal (indicative of a physiological parameter or vital sign) of the user; and
- a sweat removal device adapted to move sweat away from said contact sensor.

In a further aspect of the present invention a method for measuring a physiological signal of the user is presented, the method comprising the steps of:
- applying a wearable device comprising a contact sensor to the skin of the user, wherein the contact sensor is adapted to contact a skin of the user and adapted to measure a physiological signal (indicative of a physiological parameter or vital sign) of the user;
- measuring a first portion of the physiological signal with said contact sensor;
- removing sweat from a skin portion in contact with said contact sensor; and
- measuring a second portion of the physiological signal with said contact sensor.

Preferred embodiments of the invention are defined in the dependent claims. It shall be understood that the claimed method can have similar and/or identical preferred embodiments as the claimed wearable device, in particular as defined in the dependent claims and as disclosed herein.

As explained above, in order to get reliable measurements, the sensors of wearable devices such as wrist watches or bracelets need to make appropriate contact with the skin of the subject. Hence, the device should be preferably be strapped quite tightly. Additionally, in particular in the case of a sensor using a light-based measurement with no proper mechanism to cope with ambient light, the housing or case around the sensor should also be light tight, i.e., provide a light-tight seal with the skin of the subject, to avoid noise from external light.

The present invention is based on the idea to provide a wearable device adapted to be worn by a user that is further equipped with a sweat removal device or means for a sweat removal adapted to move sweat away from the contact sensor. The sweat removal device can be adapted to move sweat away from a skin portion where the contact sensor is adapted to contact the skin of the user. In prior art devices, sweat cannot escape the device and may accumulate between the device and the skin. This can lead to wearing discomfort and skin irritation. The inventors have found that this situation not only occurs if the wearable device is used during sports, which may lead to excessive sweating, but even during sleep sweat may accumulate underneath or in the surrounding of the contact sensor which may in turn lead to wearing discomfort and skin irritation. Hence, by providing the wearable device with a sweat removal device adapted to move sweat away from said contact sensor the wearing comfort can be improved and in particular skin irritation can be reduced.

Furthermore, it has been found that sweat accumulating between the skin and the wearable device, in particular at the contact sensor, may cause erroneous readings. For example, when using an optical measurement principle, sweat may lead to undesirable reflections that disturb the measurement. When using an impedance-based measurement, excessive sweat may provide an increased (DC) conductivity that may deteriorate a signal-to-noise ratio. The wearable device proposed herein comprising the sweat removal device may thus also enable measurements with improved accuracy and/or reliability.

A wearable device as used herein can refer to an activity tracker or activity monitor, typically wrist-worn, but may also refer to a medical device at least a part of which is worn by the user for acquiring a physiological signal of the user.

A physiological signal of the user can refer to a physiological signal indicative of a vital sign or physiological parameter of the user. For instance, a physiological signal can be indicative of a PPG measurement or bio-impedance measurement, from which at least a parameter indicative of a vital sign of the user can be derived such as, for example, a heart rate, respiration rate, blood oxygen saturation, galvanic skin response or electro-dermal activity.

A contact sensor adapted to contact a skin of the user and adapted to measure a physiological signal of the user as used herein refers to a sensor that is in direct contact with the skin, for example a heart rate sensor or other sensor that is adapted to perform an electrical and/or optical measurement on the skin of the user. In particular, the contact sensor may not be a biochemical sensor, in particular not a biochemical sensor for analyzing (a composition of) sweat. For contacting the skin of the subject, the sensor can be arranged, for example, at a side of a housing or wrist band of the wearable device facing the skin of the subject in an as-worn orientation. Hence, the contact sensor can be adapted to optically or electrically contact the skin of the user. The contact sensor is adapted to make direct contact with the skin of the user.

The proposed solution is particularly advantageous in designs, wherein the contact sensor protrudes from a housing or wrist band of the wearable device towards the skin of the user in an as-worn orientation. Hence, even in case the sweat is not completely moved to an outside of the wearable device, an advantageous effect can already be reached by moving the sweat away from said contact sensor.

In an embodiment, the contact sensor can comprise at least one of a photoplethysmography (PPG) sensor and a bio-impedance sensor. In case of a PPG sensor, the use of a sweat removal device adapted to move sweat away from said contact sensor is particularly advantageous since sweat can from a reflective film that may deteriorate the optical PPG measurement. For example, a reflection on a sweat-air-interface may be measured instead of the time-varying absorption variations within the tissue due to cardiac pulses. In case of a bio-impedance sensor, the use of a sweat removal device is particularly advantageous because a (conducting) sweat layer on the skin may deteriorate a measurement of the galvanic skin response. Hence, an improved signal-to-noise ratio for the measurement of galvanic skin response (in particular with respect to GSR peaks) can be provided. A bio-impedance sensor can also refer to a galvanic skin response (GSR) or electro-dermal activity (EDA) sensor.

Optionally, the sweat removal device can have a side or outlet exposed to air in order to allow the evaporation of the sweat that has been moved away from the contact sensor. The sweat removal device can comprise a channel. Am opening can of the channel can be arranged adjacent to the contact sensor for sucking sweat away from said contact sensor. A second opening in fluid communication with said first opening can be exposed to air to allow the evaporation of the sweat that has been moved away from the contact sensor.

Optionally, the sweat removal device can be adapted to actively move sweat away from the sensor. The sweat removal device can comprise a pump adapted to (actively) move sweat away from the contact sensor. Energy can be provided from an energy source to operate said pump.

Optionally, the sweat removal device can be adapted to move sweat away from the contact sensor by electro-wetting. The sweat removal device can comprise an electro-wetting pump. An electro-wetting pump can refer to a device adapted to move sweat (away from the contact sensor) by electro-wetting. Moving a fluid, here sweat, by electro-wetting is particularly advantageous for removing fluid in the form of droplets. One advantage of this embodiment is that also small quantities of sweat can be effectively moved away from the contact sensor. A further advantage can reside in efficient (droplet-based) sweat removal. The underlying working principles of electro-wetting, which may form the basis for providing an electro-wetting pump, are explained in Mugele et al., "Electro-wetting: From Basics to Applications", Journal of Physics: Condensed Matter, vol. 17, no. 28, 2005.

Optionally, the sweat removal device can be adapted to move sweat through a channel by electro-wetting, wherein the channel comprises a hydrophobic surface. Optionally, the sweat removal device can comprise an electro-wetting pump comprising a channel having a hydrophobic surface. For example, the channel can be a channel for transporting sweat away from the contact sensor and the channel can be made of a hydrophobic material or can have a hydrophobic coating.

On the other hand, when using a capillary pump, the sweat removal device or capillary pump may comprise a channel comprising a hydrophilic surface. A hydrophilic surface has been found to provide good performance for capillary pumps. Optionally, the sweat removal device can comprise a first channel comprising a hydrophobic surface and a second channel comprising a hydrophilic surface. The first channel can be used for moving sweat by electro-wetting whereas the second channel may be used in combination with a capillary pump.

Optionally, the sweat removal device can be adapted to move sweat away by electro-wetting can comprise an array of electrodes. The sweat removal device can be adapted to sequentially activate neighboring electrodes. In particular, a plurality of electrodes can be switched in a cascaded manner, thereby moving a droplet of sweat along with the respective activated electrodes.

Optionally, the sweat removal device can comprise a planar array of electrodes arranged adjacent to the contact sensor and arranged to face a skin of the user, when the wearable device is worn by the user. In this configuration, a plurality of electrodes may thus be provided, for example, on a lower side of a housing of the wearable device, such that a droplet between the skin of the subject and the housing can be moved away from the sensor (parallel to the electrodes and the skin of the subject) by selectively activating the electrodes and moving the droplet by electro-wetting. An advantage of this embodiment is easy cleaning and device reliability. The electrode arrangement can be cleaned easily and advantageously no channels are provided that may become clogged, for example, due to dead skin cells or debris.

Optionally, the sweat removal device can comprise a capillary pump. The capillary pump can be adapted and arranged to suck away sweat from the contact sensor. The use of the capillary pump is particularly advantageous when there is a continuous layer of fluid. Exemplary capillary pumps are described by Zimmermann et al. in "Capillary pumps for autonomous capillary systems", Lab Chip, vol. 7, pages 119-125, 2007. The wearable device can comprise a strap such as a wrist strap, and the capillary pump can be arranged on the strap, for example on a side of the strap adjacent to a housing of the wearable device. Optionally, the strap can be made of cloth or other water absorbent material. The (capillary) pump or a channel in fluid communication with the pump can be connected to the cloth. An advantage of this embodiment is that an extended region, advantageously the entire wrist band can be used for collection of the fluid. Optionally, a surface exposed to air can be further increased thereby for evaporation. The capillary pump can be in fluid communication with an opening adjacent to the contact sensor. Hence, sweat can be sucked away through said opening. Alternatively, the capillary pump can be arranged on top of a housing of the wearable device.

Optionally, the sweat removal device can comprise a combination of a capillary pump and a device adapted to move (or means for moving) sweat away from the sensor by electro-wetting. In other words, the sweat removal device can comprise a combination of a capillary pump and an electro-wetting pump. An advantage of this combination can be more efficient sweat removal. Electro-wetting provides advantageous performance for removing droplets whereas the capillary pump provides advantageous performance for removing a (connected) fluid layer. The device or means for moving sweat away from the contact sensor by electro-wetting and a capillary pump can be arranged in series. For example, the sweat removal device comprises a first electro-wetting stage in fluid communication with a second stage capillary pump. Electro-wetting can be used to collect droplets of sweat which are then further removed (after being collected, optionally forming a connected fluid layer) by the capillary pump. Thereby, efficient, in particular energy efficient, sweat removal can be provided which can be crucial for a wearable device where battery life is limited. As a further advantage, electro-wetting can be used to unclog a channel of a capillary pump. The sweat removal device may thus be adapted to unclog a channel of a capillary pump using electro-wetting, e.g. to unclog a channel filled with air bubbles.

Optionally, the wearable device can comprise a sweat sensor and a controller, wherein the controller is adapted to activate the sweat removal device based on sweat detection by the sweat sensor. An advantage of this embodiment is increased efficiency, since the sweat removal device may only be activated upon detection of sweat by the sweat sensor, i.e., when it is actually needed. In a refinement, the contact sensor adapted to contact the skin of the user and adapted to measure the physiological signal of the user may also be adapted to serve as the sweat sensor. For example, the controller may activate the sweat removal device if a DC level of a bio-impedance measurement is below a predetermined threshold. Hence, a continuous layer of sweat may provide a reduced impedance which is measured by the bio-impedance measurement.

Optionally, the sweat removal device can comprise an array of electrodes and the sweat removal device can be adapted to sequentially activate (neighboring) electrodes to move sweat by electro-wetting and the sweat removal device can be adapted to sequentially activate neighboring electrodes if an impedance (e.g. capacitance or resistance) between neighboring electrodes crosses a predetermined threshold. For example, an electrode can be activated if an impedance exceeds a predetermined threshold or is below a predetermined threshold. Hence, the electrodes of the sweat removal device can also be used as a sweat sensor thereby providing a synergistic effect. The sweat removal device can be adapted to activate a series of neighboring electrodes in a cascaded manner to move sweat by electro-wetting.

Optionally, the wearable device can further comprise a sweat analysis sensor, wherein the sweat removal device is adapted to move sweat away from the contact sensor and towards the sweat analysis sensor. Hence, a synergistic effect can be provided in that the removal of sweat from the contact sensor also guides the sweat towards the sweat analysis sensor for further analysis. The sweat analysis sensor can be adapted to determine a concentration of one of more analytes in the sweat. Hence, a composition of the sweat can be analyzed. Exemplary analytes can be one or more of glucose, lactate, sodium ion (Na⁺), potassium ion (K⁺), chloride (Cl⁻), hydrogen ion (H⁺, thus measuring pH) or any other analyte. Even if no absolute concentrations in these analytes can be given, it can be interesting for the user to see how the concentrations change over time, thereby providing a relative measurement, for example during exercising.

Optionally, the sweat removal device can be adapted to move sweat away from at least a first side and a second side of the contact sensor. The wearable device can comprise a housing. The contact sensor can be arranged at a lower side of the housing facing towards the skin of the subject, when the wearable device is worn by the user. The sweat removal device can comprise an opening arranged at a side of the contact sensor for moving sweat away from the contact sensor. Preferably, at least one first opening is arranged at a first side of the contact sensor and at least one second opening is arranged at a second side of the contact sensor. The openings are in fluid communication with the sweat removal device and adapted to move sweat away from the contact sensor.

Optionally, the contact sensor can comprise a first contact sensor portion, and a second contact sensor portion, wherein the sweat removal device is adapted to move sweat away from between said first contact sensor portion and said second contact sensor portion. For example, the first and the second contact sensor portion can be part of one heart rate sensor. In case of a PPG sensor, the first contact sensor portion can be a light source of the PPG sensor and the second contact sensor portion can be a light detector of the PPG sensor. In case of a bio-impedance sensor, the first contact sensor portion can be a first electrode of the bio-impedance sensor and the second contact sensor portion can be a second electrode of the bio-impedance sensor. By removing sweat away from in between said first and second contact sensor portions, the measurement accuracy can be improved, since in particular the direct measurement path is only exposed to a reduced amount of sweat.

### BRIEF DESCRIPTION OF THE DRAWINGS

These and other aspects of the invention will be apparent from and elucidated with reference to the embodiment(s) described hereinafter. In the following drawings:
Fig. 1 shows a perspective view of a first embodiment of a wearable device;
Fig. 2 shows a side view of an embodiment of a wearable device;
Fig. 3 shows a bottom view of an embodiment of a wearable device;
Fig. 4 shows a bottom view of a further embodiment of a wearable device;
Fig. 5 shows a schematic diagram of a partial cross-section of a wearable device;
Fig. 6 shows a schematic diagram of and electro-wetting pump;
Fig. 7 shows a schematic diagram of another embodiment of a contact sensor and a sweat removal device;
Fig. 8 shows a perspective view of a further embodiment of a wearable device; and
Fig. 9 shows a schematic flow chart of a method according to an aspect of the present disclosure.

### DETAILED DESCRIPTION OF THE INVENTION

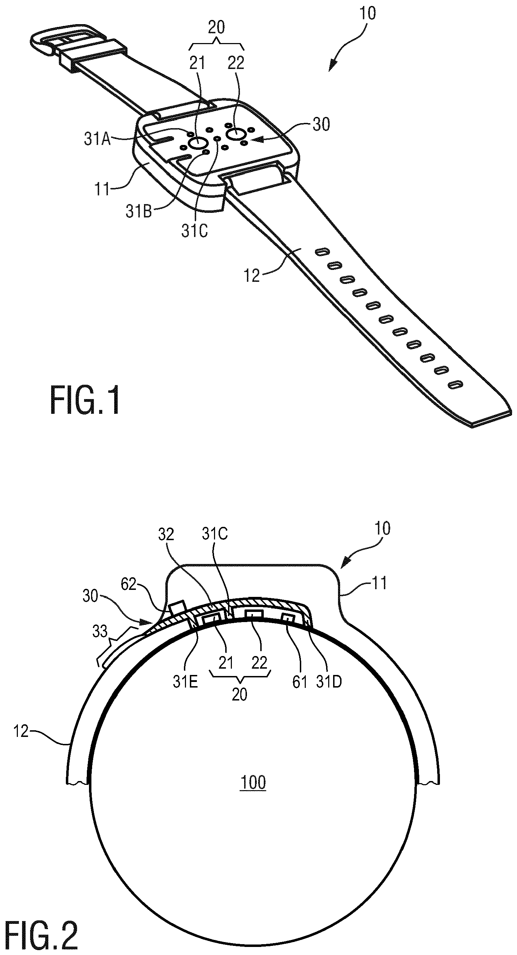

Fig. 1 schematically shows a first embodiment of a wearable device according to an aspect of the present disclosure. The wearable device is therein denoted in its entirety by reference numeral 10. The exemplary wearable device 10 of this embodiment is implemented in form of a wrist watch or activity tracker comprising a housing 11 and a wrist band 12. The housing of the wearable device 10 can comprise battery, electronics, a communication interface, e.g. for communication with a smartphone, and a display. Alternative embodiments can refer to a wearable device adapted to be attached to a skin of the subject, for example, using a chest band, embedded in clothing or using an adhesive.

With photoplethysmography (PPG), bio-impedance or capacitive technology, contact sensors adapted to contact a skin of the user and adapted to measure a physiological signal indicative of a vital sign (such as the heart rate of the user) can be integrated in wearable devices such as wrist watches or bracelets. In order to get reliable measurements, the sensors need to make appropriate contact with the skin and the device should preferably be strapped quite tightly. Additionally, in particular in case of light-based sensor with no proper mechanism to cope with ambient light, the case or housing around the sensor should also be light tight to avoid noise from external light. Since sweat cannot escape in prior art devices, it accumulates between the device and the skin, which may lead to wearing discomfort and skin irritation. This is in particular a problem in situation when the wearable device is used during sports, which leads to excessive sweating. However, it has been found that even during non-strenuous activities and even during sleep sweat may accumulate and lead to wearing discomfort and skin irritation. Next to wearing discomfort and skin irritation, sweat accumulating between the skin and the device might cause in accurate readings.

The wearable device 10 comprises a contact sensor 20 adapted to contact a skin of the user and adapted to measure a physiological signal (indicative of a physiological parameter or vital sign) of the user. In the embodiment shown in Fig. 1, the contact sensor 20 can be a bio-impedance sensor comprising a first electrode 21 and a second electrode 22.

The wearable device 10 further comprises a sweat removal device 30 adapted to move sweat away from said contact sensor 20. The sweat removal device 30 can comprise a pump that is in fluid communication with one or more openings 31A-31C arranged for move sweat away from said contact sensor. The openings 31A-31C can be arranged adjacent to the contact sensor 20 and arranged to face a skin of the subject, when the wearable device 10 is worn by the user. Advantageously, the sweat removal device 30 is adapted to move sweat away from at least a first side and a second side of the contact sensor 20. For example, a first opening 31A can be provided at a first side of the contact sensor 20 and a second opening 31B can be provided at a second side of the contact sensor 20 for sucking away sweat from the contact sensor.

Advantageously, for the case that the contact sensor 20 comprises a first contact sensor portion, here a first electrode 21 of a bio-impedance sensor, and a second contact sensor portion, here a second electrode 22 of the bio-impedance sensor, the sweat removal device 30 can be adapted to move sweat away from between said first contact sensor portion 21 and said second contact sensor portion 22. In a given example one or more openings 31C can thus be provided between the first contact sensor portion 21 and the second contact sensor portion 22.

Fig. 2 shows a side view of a wearable device 10 adapted to be worn by a user 100. In the shown embodiment, the wearable device is adapted to be worn around a wrist of the user 100. The wearable device 10 again comprises a housing 11 and a wrist band 12. In the embodiment shown in Fig. 2, the sweat removal device is adapted to move sweat away from said contact sensor 20, in particularly to move sweat from the skin of the subject 100 in proximity of or in the surrounding of the contact sensor 20 away from the contact sensor 20. The sweat removal device 30 can comprise one or more openings 31C-31E that are in fluid communication with a pump 33, here in form of a capillary pump 33 that is connected to said openings via one or more channels 32. In order to provide good sweat removal performance it is advantageous if the sweat removal device 30 is adapted to move sweat away from at least a first side and a second side of the contact sensor 20, for example via the openings 31D and 31E. Furthermore, if the contact sensor 20 comprises a first contact sensor portion, here first electrode 21, and a second contact sensor portion, here second electrode 22, the sweat removal device is preferably adapted to move sweat away from between said first contact sensor portion and said second contact sensor portion, for example via an opening 31C arranged in between said first and said second contact sensor portion.

Optionally, the wearable device 10 can comprise a sweat sensor 61 and a controller, wherein the controller is adapted to activate the sweat removal device 30 based on sweat detection by said sweat sensor 61. Optionally, the contact sensor 20 may be configured to serve as the sweat sensor 61. For example, an electrode 21 or 22 of the contact sensor 20 can be used in sweat detection for example by a capacitive measurement. In addition or in the alternative, the measurement of a bio-impedance sensor can be evaluated. The bio-impedance sensor can be adapted to evaluate an impedance and activate the sweat removal device 30 based on the measured impedance. For example a sweat layer can lead to a reduced resistance between the first electrode 21 and the second electrode 22 of the contact sensor 20 in form of a bio-impedance sensor. The wearable device may thus be adapted to activate the sweat removal device 30 if the impedance (e.g. DC resistance) is below a predetermined threshold.

Optionally, the wearable device 10 can further comprise a sweat analysis sensor 62, wherein the sweat removal device 30 is adapted to move sweat away from the contact sensor 20 and towards the sweat analysis sensor 62. In the shown example, the sweat analysis sensor 62 is coupled to a channel 32 of the sweat removal device 30. The pump 33 of the sweat removal device thus pumps the sweat away from the skin of the user 100 through one or more openings 31 through the channels 32 towards the sweat analysis sensor 62. The sweat removal device 30 may optionally further pump the sweat to the outside e.g. for evaporation. A capillary pump 33 may for example be implemented as described in the afore-mentioned paper by Zimmermann et al.

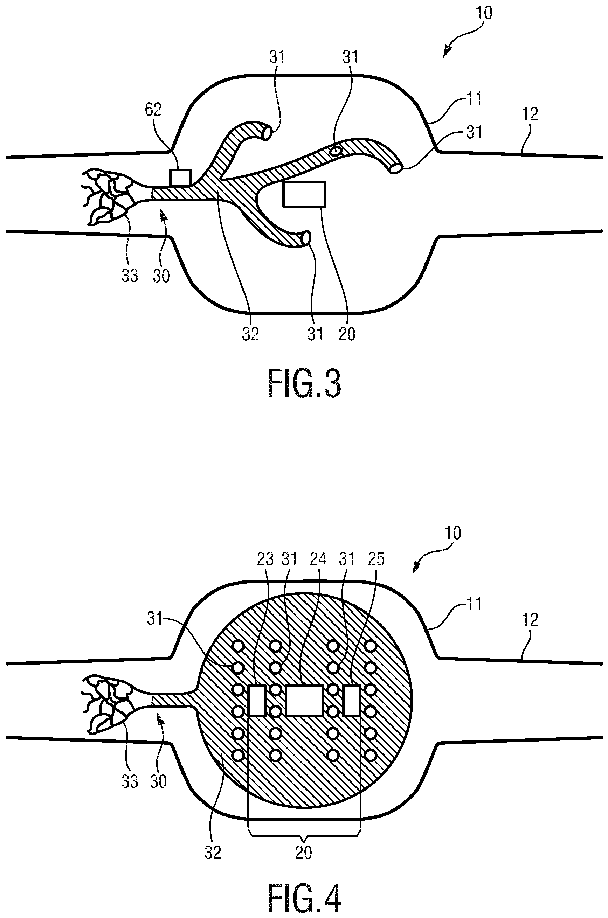

Fig. 3 and Fig. 4 show schematic bottom views of two different embodiments of wearable devices 10. The embodiment shown in Fig. 3 shows a channel 32 that is small in two dimensions. The embodiment shown in Fig. 4 shows a channel that is only small in one dimension.

In the embodiment shown in Fig. 3, the sweat removal device can comprise a pump, here in form of a capillary pump 33, one or more channels 32 and one or more openings 31. The pump is in fluid communication with the openings 31 via channels 32 to move sweat away from the contact sensor 20. The channel 32 can have several branches. Each branch can be connected to one or more openings. For example, two openings can be provided as shown to the top right of the optical sensor 20.

In addition or in the alternative, the channel may not have a closed circular shape. The channel can comprise an opening in form of a slit or, more generally speaking, the channel can be arranged to be open towards the skin of the subject, when the wearable device is worn by the user. For example, the channel can have a semi-circular form open towards the skin of the subject. The channel may form a recess in the lower side of a housing or wrist band of the wearable device. Sweat can be actively moved away from the contact sensor 20 by a pump connected to said channel 32.

Fig. 4 shows a further embodiment of a wearable device 10. The channel 32 can be formed as a hollow space within the housing 11 of the wearable device 10 comprising one or more openings 31 on the bottom side of the housing 11 to make connections with the channel 32 for moving sweat away from the contact sensor 20. The channel 32 is in fluid communication with the pump 33, here a capillary pump. Other pumps can be used such as an electro-wetting pump 33' operating based on electro-wetting to move (droplets of) a fluid.

In the shown embodiment in Fig. 4, the contact sensor 20 is a photoplethysmography (PPG) sensor. The PPG sensor can comprise at least one light source 23 and a photodetector 24. The PPG sensor can comprise a first contact sensor portion 23, for example comprising the light source of the PPG sensor, and a second contact sensor portion 24, for example comprising the detector of the PPG sensor. Optionally, the device can comprise a second light source 25. The second light source can optionally be implemented as yet another contact sensor portion 25. For example, the first light source can be adapted to emit light at a first wavelength such as red light whereas the second light source can be adapted light at a second wavelength, for example infrared light, so as to enable blood oxygen saturation measurements (SpO₂ measurements).

Figs. 3 and 4 show a pump 33, here in form of a capillary pump, on one side of the strap 12. Other embodiments may also have a (capillary) pump and a respective connecting fluid channel 32 on the other side of the strap and/or have the (capillary) pump on top of the housing 11. Optionally, the pump 33 may have a side exposed to air in order to allow evaporation of the fluid. The capillary pump could also be absent, e.g. when electro-wetting is used as a mechanism for active fluid displacement.

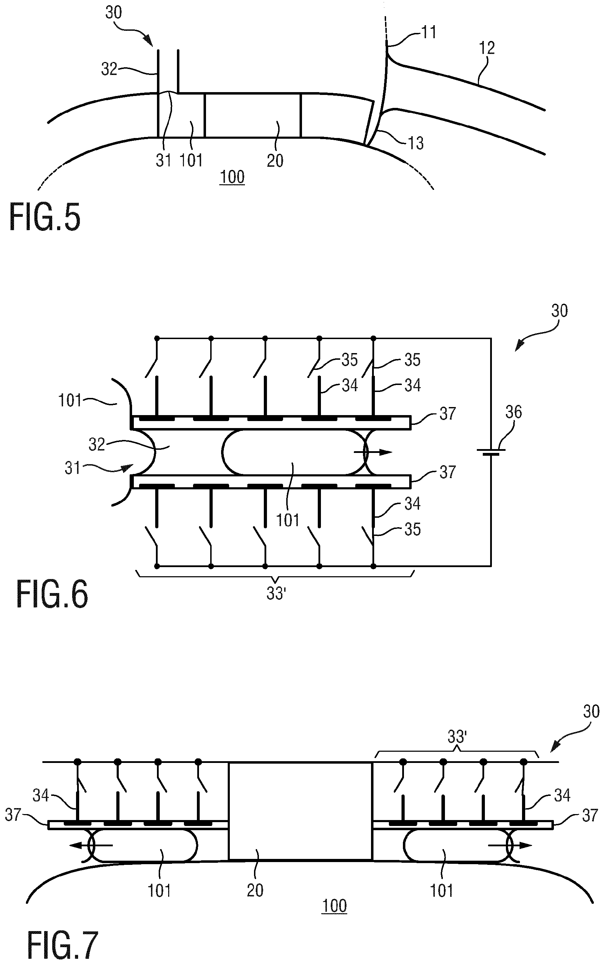

Fig. 5 illustrates the case where sweat 101 accumulates next to the contact sensor 20. The contact sensor 20 may protrude from the housing 11 of the wearable device 10. As shown in Fig. 5, the wearable device 10 can comprise a housing 11 and a wrist strap 12. On a lower side of the housing facing the skin of the subject 100, an optical shield 13 may be provided that prevents stray light from entering the device from the side. This is particularly advantageous when using a contact sensor 20 operating based on an optical measurement principle such as PPG. However, such an optical shield 13 at the same time prevents sweat 101 from leaving the device such that sweat 101 may accumulate and lead to wearing discomfort and skin irritation. According to the solution proposed herein, a sweat removal device 30 is provided. In the shown embodiment, the sweat removal device 30 comprises an opening 31 that is connected to a channel 32 to move the sweat 101 away from the sensor 20. As described in the previous embodiments, the channel 32 can be connected to a pump such as a capillary pump. Optionally, the sweat can be moved away from the sensor by electro-wetting as will be explained with reference to the examples shown in Figs. 6 and 7.

Figs. 6 and 7 illustrate the principle of electro-wetting to displace a fluid or a droplet such as sweat 101 from the skin of the subject 100. According to an another aspect of the present disclosure, a wearable device adapted to be worn by a user is presented, the wearable device comprising a fixing device for fixing the wearable device to the user, the wearable device having a lower side for contacting a skin of the user when worn, the wearable device further comprising an electro-wetting pump adapted to move sweat away from at least a portion of said lower side for contacting a skin of the user when worn by electro-wetting. According to a further aspect of the present disclosure, a wearable device adapted to be worn by a user is presented, the wearable device comprising a sensor and an electro-wetting pump adapted to move sweat away from the sensor and/or towards the sensor. Hence, the electro-wetting pump can also be used independent of a contact sensor adapted to contact the skin of the user and adapted to measure a physiological signal of the user. The electro-wetting pump can also be used in combination with a sweat analysis sensor, in particular adapted to determine a concentration of one or more analytes in the sweat. Advantageously, in the case of a wearable device 10 comprising a contact sensor such as a PPG sensor, the electro-wetting device can be adapted to move the sweat from a surrounding of the sensor away from the sensor. In an alternative embodiment, the electro-wetting device can be used to move the sweat to a sweat analysis sensor or to a chamber for analysis.

Referring to the embodiment shown in Fig. 6, the sweat removal device 30 can comprise an electro-wetting pump 33', i.e., a pump or device adapted to move sweat 101 by electro-wetting. The sweat removal device 30 can comprise an opening 31 where a droplet of sweat 101 can be sucked in, a channel 32 in fluid communication with the opening and the electro-wetting pump 33'. The electro-wetting pump 33' can comprise an array of electrodes 34. The electrodes 34 can be controlled independently, for example, by means of switches 35 as illustrated in Fig. 6. In the shown embodiment, the electrodes 34 are separated from an inner part of the channel 32 through a dielectric layer 36. The electrodes 34 can be activated by closing the switches 35 and thus applying a voltage from a voltage source 36 to the respective electrodes.

The dielectric layer 37 can advantageously be intrinsically hydrophobic or can be coated with a hydrophobic material (typically Teflon). Thus, in a non-active state, the fluid, here a droplet of sweat 101, is repelled due to the hydrophobicity of the surface 37. However, when a voltage V is applied at an electrode 34, preferably between a pair of two electrodes facing each other, the contact angle of the fluid 101 can change resulting in a hydrophilic behavior. The resulting effect is the movement of the fluid 101 toward the hydrophilic part as illustrated by the arrow in Fig. 6.

In order to control the movement of the fluid, several methods can be implemented. In a basic embodiment, the device can be adapted to continuously switch the electrodes 34 in a cascaded manner. Hence, the "hydrophilic part" moves along the channel 32, thereby transporting the droplet of sweat 101.

In an advantageous refinement, one or more of the electrodes 34 of the electro-wetting pump 33' can be used for both sensing and actuating. The presence of fluid can be detected through e.g. a drop in impedance (resistance) between (neighboring) electrodes 34 of the electro-wetting pump 33'. Upon detection of an impedance change between (neighboring) electrodes 34 the appropriate electrodes can be activated e.g. by applying a higher voltage, to create the fluid motion. Advantageously, the electro-wetting pump can be adapted to automatically start/stop when detecting fluid at the electrode near the opening 31 / near the skin and to automatically generate a cascaded switching to move the droplet along the channel 32. The operation can continue until the fluid is not detected anymore in the channel 32 or after a predetermined time.

A voltage required for operation of an electro-wetting pump is typically 10-20 volts. This can be achieved in a watch using an operational amplifier. A battery in a watch typically supplies about 2.5 volt. The power consumption for actuating a drop is low, in particular ≤ 0.1 mW. Optionally, for example in a medical device, a higher voltage battery may be used. A channel side (cross-section) x of a channel 32 of a sweat-removal device can be 0.1 mm ≤ x ≤ 2 mm, in particular 0.2 mm ≤ x ≤ 1 mm, in particular 0.3mm ≤x ≤ 0.5 mm.

Fig. 7 shows an alternative embodiment of an electro-wetting pump 33' operating in a single-sided configuration. This can also be referred to as an open configuration. The sweat removal device 30 can comprise an array of electrodes and the sweat removal device 30 can be adapted to sequentially activate (neighboring) electrodes 34 to move sweat by electro-wetting, as exemplarily illustrated by moving the droplet of sweat 101 in direction of the arrow. The sweat removal device 30 comprises a planar array of electrodes 34 arranged adjacent to the contact sensor 20 and arranged to face a skin of the subject 100, when the wearable device is worn by the user. Hence, instead of providing a closed channel the lower side of the housing 10 or of the wrist band 12 of the wearable device, i.e. the side facing the skin of the subject 100, can comprise an electro-wetting pump configured to move sweat away from the contact sensor. Compared to the embodiment shown in Fig. 7 wherein electrodes 34 can be arranged at two sides of the channel 32, the embodiment shown in Fig. 7 can also be referred to as a wearable device comprising a single-sided electro-wetting pump 33'.

Hence, the droplet sweat 101 can be moved away from the contact sensor 20 along the lower side of the housing 11 of the wearable device 10.

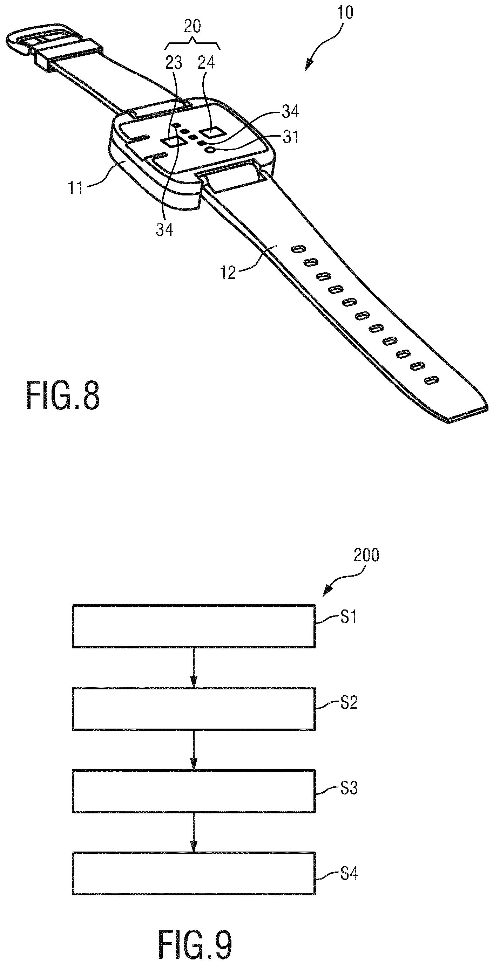

Fig. 8 shows an exemplary perspective view of a wearable device 10 comprising an electro-wetting pump comprising a plurality of electrodes 34. In the shown example, the contact sensor 20 can be a PPG sensor comprising a light source 23 and a detector 24. In the shown embodiment, the electro-wetting pump is adapted to move sweat away from between the PPG light source 23 as a first contact sensor portion and the PPG detector 24 as a second contact sensor portion.

Optionally, the sweat removal device 30 can comprise a combination of a capillary pump and a device for moving sweat away from the contact sensor 20 by electro-wetting. For example as shown in Fig. 8, a plurality of electrodes 34 can be provided for moving the sweat towards an opening 31 which is in fluid communication with a capillary pump. Hence, the electro-wetting pump and the capillary pump can be arranged in series comprising a first electro-wetting stage in fluid communication with a second-stage capillary pump. For example droplets of sweat can be collected via electro-wetting which is then further removed by the capillary pump through opening 31. Furthermore, in case of large amounts of sweat, the capillary pump can be advantageous for removing a closed fluid layer from underneath the wearable device 10.

Fig. 9 shows a flow chart of a method 200 for measuring a physiological signal (indicative of a physiological parameter or vital sign) of the user. In the first step S1, the wearable device comprising a contact sensor is applied to the skin of the user, wherein the contact sensor is adapted to contact a skin of the user and adapted to measure a physiological signal of the user. In a second step S2, a first portion of the physiological signal is measured with said contact sensor. In a third step S3, sweat is removed from a skin portion in contact with said contact sensor. The sweat can be underneath the contact sensor i.e. between the contact sensor and the skin of the subject or in the periphery or surrounding or in proximity of the contact sensor. In a fourth step S4, a second portion of the physiological signal is measured with said contact sensor. The wearable device can comprise a sweat removal device adapted to move sweat away from the contact sensor. Hence, in step S3 the sweat can be removed from a skin portion in contact with the contact sensor using the sweat removal device adapted to move sweat away from the contact sensor. The sweat removal device can be a sweat removal device comprising at least some of the features described above.

In conclusion, the wearable device and method as presented herein, enable an improved wearing comfort, in particular by reducing skin irritation due to sweat. Furthermore, the measurement accuracy can advantageously be improved since the removal of sweat can enable more accurate optical measurements due to reduced reflections and/or more accurate bio-impedance measurements since an impedance-influence due to a sweat layer on top of the skin can be reduced.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. Wearable device (10) adapted to be worn by a user (100), the wearable device (10) comprising:
- a contact sensor (20) adapted to contact a skin of the user (100) and adapted to measure a physiological signal of the user; and
- a sweat removal device (30) adapted to move sweat away from said contact sensor (20).

2. Wearable device as claimed in claim 1, wherein the contact sensor (20) comprises at least one of a photoplethysmography (PPG) sensor and a bio-impedance sensor.

3. Wearable device as claimed in claim 1, wherein the sweat removal device (30) is adapted to actively move sweat away from the contact sensor (20).

4. Wearable device as claimed in claim 1, wherein the sweat removal device (30) is adapted to move sweat away from the contact sensor by electro-wetting.

5. Wearable device as claimed in claim 4, wherein the sweat removal device (30) is adapted to move sweat through a channel (32) by electro-wetting, wherein the channel (32) comprises a hydrophobic surface (37).

6. Wearable device as claimed in claim 4, wherein the sweat removal device (30) comprises an array of electrodes (34) and wherein the sweat removal device (30) is adapted to sequentially activate neighboring electrodes (34) to move sweat by electro-wetting.

7. Wearable device as claimed in claim 6, wherein the sweat removal device (30) comprises a planar array of electrodes (34) arranged adjacent to the contact sensor (20) and arranged to face a skin of the user (100), when the wearable device (10) is worn by the user (100).

8. Wearable device as claimed in claim 1, wherein the sweat removal device (30) comprises a capillary pump.

9. Wearable device as claimed in claim 8, sweat removal device (30) comprising a combination of a capillary pump (33) and a device (33')adapted to move sweat away from the contact sensor (20) by electro-wetting.

10. Wearable device as claimed in claim 1, further comprising a sweat sensor (61) and a controller, wherein the controller is adapted to activate the sweat removal device (30) based on sweat detection by the sweat sensor (61).

11. Wearable device as claimed in claim 6, the sweat removal device (30) is adapted to sequentially activate neighboring electrodes (34) if an impedance between neighboring electrodes crosses a predetermined threshold.

12. Wearable device as claimed in claim 1, further comprising a sweat analysis sensor (62), wherein the sweat removal device (30) is adapted to move sweat away from the contact sensor (20) and towards the sweat analysis sensor (62).

13. Wearable device as claimed in claim 1, wherein the sweat removal device (30) is adapted to move sweat away from at least a first side and a second side of the contact sensor (20).

14. Wearable device as claimed in claim 1, wherein the contact sensor (20) comprises
- a first contact sensor portion (21, 23), in particular a light source of a PPG sensor (23) or a first electrode of a bio-impedance sensor (21), and
- a second contact sensor portion (22, 24), in particular a detector of the PPG sensor (24) or a second electrode (22) of the bio-impedance sensor,
wherein the sweat removal device (30) is adapted to move sweat away from between said first contact sensor portion (21, 23) and said second contact sensor portion (22, 24).

15. Method for measuring a physiological signal (indicative of a vital sign) of the user (100), the method comprising the steps of:
- applying a wearable device (10) comprising a contact sensor (20) to the skin of the user, wherein the contact sensor (20) is adapted to contact a skin of the user (100) and adapted to measure a physiological signal (indicative of a vital sign) of the user (100);
- measuring a first portion of the physiological signal with said contact sensor (20);
- removing sweat from a skin portion in contact with said contact sensor (20); and
- measuring a second portion of the physiological signal with said contact sensor (20).
